# EUROPEAN PATENT SPECIFICATION

(11) **EP 1 493 396 B1**
(45) Date of publication and mention of the grant of the patent: **23.09.2009**
(21) Application number: 04004807.6
(22) Date of filing: 02.03.2004
(51) Int. Cl.: A61B 17/34

(54) **Device for mounting medical instruments**
Vorrichtung zum Einbringen medizinischer Instrumente
Dispositif pour introduire des instruments médicaux

(30) Priority: 30.06.2003 RU 2003119287
(43) Date of publication of application: 05.01.2005
(73) Proprietor: Shkarubo, Alexei Nikolaevich, 129278, Moscow (RU); GRIGORIEV, Anatoly Ivanovich, 129075 Moscow (RU)
(72) Inventor: Shkarubo, Alexei Nikolaevich, 129278, Moscow (RU); GRIGORIEV, Anatoly Ivanovich, 129075 Moscow (RU)
(74) Representative: HOFFMANN EITLE

(56) References cited:
- RU-C- 2 160 058
- US-A- 5 827 221
- US-B1- 6 302 875

## Description

### Field of The Invention

The invention relates to medical technique, namely surgical instruments and can be used in neurosurgery, anaesthesiology, endovascular surgery, orthopedic surgery and in other fields of medicine.

### Description of The Prior Art

Known in the art is a device for arranging medical instruments into the epidural space of the spinal cord, with which use is made of an introductor having a semirigid tubular axis with a through central lumen, a curved form-remembering distal end and a proximal end connected to a fitting. A straight needle is inserted into the central lumen of the introductor's axis up to the end of the lumen followed by said introductor with said needle being inserted through the skin into the epidural space of the spinal cord. On removal of the needle from the central lumen of the introductor's axis, its distal end again assumes the curved shape. A medical instrument is inserted into the epidural space through the free central lumen of the introductor's axis (Patent US N 5215105, cl. A 61 B 17/00, 1989 /I/). However, the described device and method for its employment are based on a subjective method of establishing admission into the epidural space - "loss-of-resistance" method - or, to be more exact, on application of this method, the real danger of perforation of the dura mater remains, with the introductor being with the needle, and also after the needle has been withdrawn, the introductor's form-remembering metal end can perforate the dura mater.

It is known to instal medical instruments, a catheter in particular, into the epidural space of the spinal cord with the aid of a Tuohy needle, using endoscopic control (epiduroscopy), i.e. an endoscope is introduced parallel to the needle, which is helpful in controlling a location of the needle's proximal portion in the epidural apace (Blomberg R.G. Technical advantages of the paramedian approach for lumbar epidural puncture and catheter introduction // Anaesthesia, 1988, Vol. 43, P. 837-843 /2/. The catheter is then installed through the needle and the moment of installation undergoes endoscopic control. A disadvantage of this particular step is a considerable complication and rise in price of the mounting procedure of medical instruments into the epidural space of the spinal cord and, along with this, the traumatism of the procedure grows significantly, the time required for manipulations is increased many a time, and the likelihood is enhanced for inflammatory complications as a result.

Besides this, at the time of bleeding from the venae of the postero - external venous plexus ( posterior epidural space) it is not practically possible to realize endoscopic control over reliability of the mounting of a medical instrument into the epidural space of the spinal cord (the bleeding from the afore-said venae frequently accompanies epidural puncture).

Known is a device of the firm "B-Braun" (B-Braun Melsungen AG") for mounting a medical instrument into the epidural space (Tezlaf D.E. Spinal, epidural and caudal anaesthesia. Section III. - Pages 273-319 // In the book by J. Edward Morgan, Jr., Magid S. Michael. - Clinical Anaesthesiology. - Binom Moscow, Nevsky Dialekt Saint Petersburg. - 1998. - Page 430 /31 /. Said device comprises: a Tuohy needle used as a guide of the medical instrument, a catheter, an epidural flat filter and a syringe used as the body. The known device may be used also for mounting the cathether into the subdural space of the spinal cord.

A disadvantage of the device of the firm "B-Braun" is the fact that while mounting a catheter or electrode into the epidural space of the spinal cord, use is made of highly subjective methods for verifying admission into the epidural space - "loss-of-resistance" method and "hanging drop" method; in other words, there remains the real danger of perforation of said spinal cord dura mater.

Known in the art is a device for localizing the proximal portion of a needle of the Tuohy type in the epidural space of the spinal cord, comprising a Macintosh balloon indicator (Bromage Ph. R. Epidural analgesia. // W.B, Saunders Company. Philadelphia - London-Toronto. - 1978. - Page 746 /4/). However, the method is based on a change (drop) in the pressure in the epidural space of the spinal cord. The Macintosh balloon indicator is inflated and put on a needle connector and is reduced in volume, with the proximal portion of the needle getting into the epidural space of the spinal cord. As a matter of fact, this is the same method thus far described and based on the "loss-of-resistance" and "hanging drop" principles, i.e. there remains the real danger of perforation of the dura mater of the spinal cord.

Also, its advantage is the fact that with a medical instrument installed by hand, particularly a catheter, great is the likelihood to damage the catheter itself by the bevel of the proximal portion of the needle /4/.

The best results in mounting instruments into the epidural or subdural space of the spinal cord are given by a special device (Patent RF (RU) N 2160058, cl. A61B 17/00, 17/34, 2000 /5/, taken as a prototype, comprising a body with an instrumental guide, a means for the preliminary fixation of a medical instrument, a trigger with a depth scale applied onto the body and a means for delivering the medical instrument in the form of telescopic tubes arranged inside the body, a first of which is immovably coupled with the body, while a second one is movable, spring-loaded, mounted with its end portion in the end face guide of the body and has a detent for retaining the medical instrument, and an arresting device provided in a slot of the body with a faculty of longitudinal movement and cooperation with the end face of the slot, the trigger is disposed on the body with a capability of movement, fixation and interaction with the arresting device to specify a depth for the medical instruments to be installed.

Device /5/ ensures the automatic atraumatic installation of medical instruments (catheter, electrode) into the epidural or subdural space of the spinal cord for a specified (regulated) depth, albeit the stability of such an installation is not enough and, as so, a surgeon should be more attentive, to manipulations to be performed. The reason behind the insufficient stability of a conventional device is the impact interaction of a body slot end face and a movable arresting device mounted in said slot. Also, installation of the medical instruments into the epidural space sequentially after flaval ligament puncture and into the subdural space - after puncturing the dura mater should be carried out in a short interval and have the possibility to be regulated, which renders the percussion mechanism of the device a most rational one. Nevertheless, the present device has a restricted region of application.

### Summary of The Invention

The technical task being solved in the present invention is to create a universal device contributing to the stable mounting of medical instruments into the epidural or subdural space of the spinal cord and also into other cavities and hollow organs, say, vessels (arteries and venae). With that so, the technical result of the present invention resides in improving convenience in operation and the contact of working elements intended for this device with the tissues and organs of a patient and also in a simple-to-use design thereof.

The task set is solved by a device for installing medical instruments, comprising a body with an instrumental guide, a means for preliminarily fixing a medical instrument, a trigger with a depth scale applied on the body and a means for delivering the medical instrument in the form of telescopic tubes arranged within the body, a first of which is immovably coupled with the body and a second one is movable, spring-loaded, loaded, mounted with its end portion in the end face guide of the body and has a detent for retaining the medical instrument, and an arresting device mounted in the body's slot with freedom to longitudinally move and cooperate with the end face of the slot; and the trigger is disposed on the body with a faculty of movement, fixation and interaction with the arresting device to specify a depth for the medical instruments to be installed. And the minimum insertion depth of the movable tube into the stationary tube is no less than one-half the outer diameter of the movable tube, and the distance between the end face guide of the body and the end portion of the stationary tube oriented to said end face guide is from 2 to 25 outer diameters of the movable tube.

In particular cases of its embodiment or use, a device may comprise a trigger configured as a collar and a double-arm spring-loaded lever, hinge jointed with the collar having an adjusting screw, while an arm of said lever has a surface for cooperation with an arresting device. An instrumental guide is a hollow needle, for example, in the form of a Tuohy needle, and connected with a body via a connector and cone congruent thereto, provided on the first tube. The body may have a cap disposed on its end on the side of the instrumental guide and retaining a medical instrument delivery means in the body, and a medical instrument preliminary fixation means with a detent is composed of spring grips of one-sided and/or double-sided action.

The cause-and-effect relationship of the listed features with the technical result of the invention, as being claimed and as set forth in the application, is expressed in that a body with an instrumental guide, a medical instrument preliminary fixation means, a trigger with a depth scale applied on the body and a medical instrument delivery means in the form of, telescopic tubes arranged inside the body, a first of which is immovably coupled with the body while a second one is movable, spring-loaded, mounted with its end portion in the body's end face guide and has a detent for retaining the medical instrument, and an arresting device provided in a slot of the body with freedom to longitudinally move and cooperate with the end face of the slot, and the trigger is disposed on the body with a faculty of movement, fixation and cooperation with the arresting device to specify a depth of placing the medical instruments - provide the automatic atraumatic installation of medical instruments (catheter, electrode, to mention just few) into the different spaces and hollow organs of a human host: the epidural or subdural space of the spinal cord, vessels (arteries, venae), various hollow organs (joint cavity etc.) for a specified (regulated) depth.

Also, stability of such an installation being responsible for the convenience of work and improvement of the contact of working elements of a device with the tissues and organs of a patient undergoing an operative procedure is ensured by a ratio of the dimensions of its composites which exert an influence on a mounting process to the utmost.

Interaction with the organs and tissues of a patient, of the working parts of a device depends on a kind of tissue, wherein an instrument is inserted, and also on the types and size of instruments introduced into the tissues. Because of the reasons listed, it is not possible to apply the experience of other mechanisms to said device, which are normally created for specific surgical operations and instruments of one type.

Investigations go to show that in the claimed device, the minimal portion of a movable tube within a stationary tube should be no less than one - half the outer dia. of the movable tube and the spacing between the end face guide of a body and the end portion oriented thereto of the stationary tube should be from 2 to 25 outer diameters of the movable tube. A failure to observe said relationships does not provide the opportunity to obtain, on the one hand, the required coaxial alignment of said movable and said stationary tubes and, as a consequence, the stable travel of an instrument and provide, on the other hand, the guaranteed penetration of the instrument into tissues of different density. For example, in cases where the minimal portion of the movable tube within the stationary tube is less than one-half the outer dia. of the movable tube, the latter repeatedly proves to be out of alignment. With the distance between the end face guide of the body and the end portion oriented thereto of the stationary tube being less than two outer diameters of the movable tube, the instrument has no impulse required for penetration into the tissues. When the distance between the end face guide of the body and the end portion oriented thereto of the stationary tube is more than 25 outer diameters of the movable tube, the misalignment of the tubes is renewed.

Implementation of a means for preliminarily fixing a medical instrument and a detent for retaining the medical instrument with freedom to cooperate with the medical instrument inserted from one and/or the other end of a body ensures the convenience of work with the device.

Simplicity of a construction is attributable to the features disclosing said construction in particular cases of its embodiment or use (cf. the dependent claims).

### Brief Description Of The Drawings

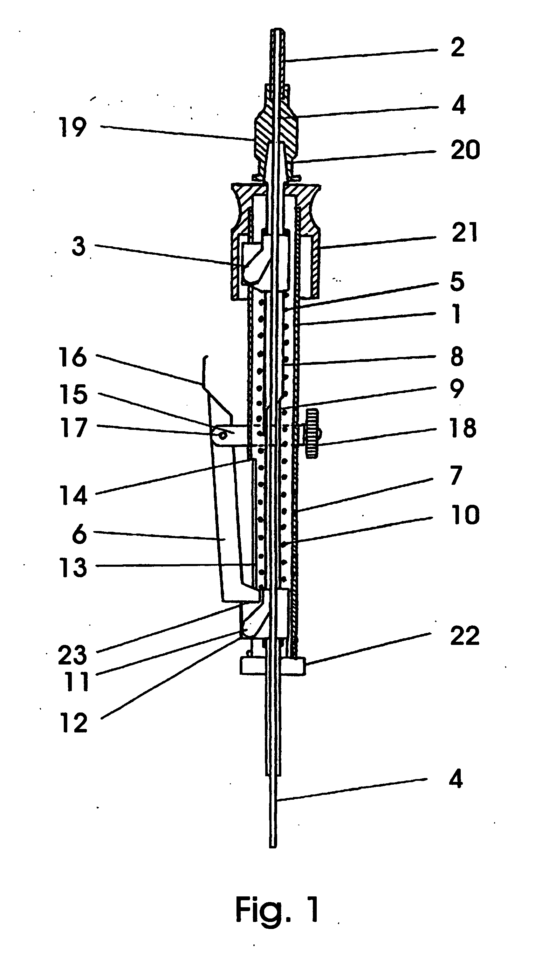

The invention will now be described in detail with reference to the accompanying drawing (Fig. 1) illustrating a device in section for mounting medical instruments, the device is shown in an operating position, with a medical instrument mounted therein (catheter, electrode etc.); Fig 2 - device, a general view, and a step of flaval ligament puncture with the aid of the device; Fig.3 - device, a general view, and a medical instrument being mounted (catheter, electrode etc.) into the epidural space of the spinal cord after flaval ligament puncture; Fig. 4 - device, a general view, and a medical instrument being installed (catheter) into the subdural space of the spinal cord; Fig. 5 - device, a general view, and a medical instrument being installed (catheter) into a vessel cavity (arteries, venae).

### Detailed Description Of The Invention

A device of the type used for mounting medical instruments comprises a body I being a hollow cylinder, an instrumental guide 2, a means 3 for preliminarily fixing a medical instrument 4, a means 5 for delivering a medical instrument, and a trigger 6 with a depth scale 7 applied onto the body I, which indicates a depth of inserting a catheter (electrode or another medical instrument): 5 mm, 10 mm, 15 mm and 20 mm. The medical instrument 4 delivery means 5 is implemented in the form of telescopic tubes 8 and 9 arranged inside the body I. The tube 8 is securely coupled with the body I , with the preliminary fixation means 3 of the medical instrument 4 attached thereto. The tube 9 is movable, spring-loaded with a spring 10, has a detent 11 for retaining the medical instrument 4 in its cavity and is provided with an arresting device 12 disposed in a slot 13 of the body I with a faculty of longitudinal travel and interaction with an end face 14 of the slot 13. The spring 10 connecting the telescopic tubes 8 and 9 provides for the tube 9 (movable) traveling into the tube 8 (stationary) thereby to provide the movement of the medical instrument 4, arranged inside the telescopic tubes, in a proximal direction. The trigger 6 is disposed on the body I with a capability of travel, fixation and cooperation with the arresting device 12.

The trigger 6 is configured as a collar 15 and a double-arm lever 16, hinge 17. jointed with the collar 15 (can be embodied as a spring-axle). Said collar also has an adjusting screw 18, and an arm of the spring- - loaded double-arm lever 16 has a surface 23 for interaction with the arresting device 12 to set a depth of installing the medical instruments ( catheter, electrode etc.).

The instrumental guide 2 of a device can be made as a hollow needle and connected with the body I by means of a connector 19 and a cone 20 congruent thereto provided on the first tube 8. The hollow needle used may be represented by a Tuohy needle.

The body I has a cap 21 disposed on its end on the side of the instrumental guide 2 and holding the delivery means 5 of the medical instrument 4 in the body I, the latter further has an end face guide 22 for the second tube 9. The preliminary fixation means 3 of the medical instrument 4 is embodied as a spring grip of one-sided action, just like the detent 11, which may have a similar construction.

The device is used in the following manner. After preparation of a patient and sterilization of the device (the body I of the device may be made of plastic material, and the device may further be included in a sterile package for one application together with the medical instrument 4, for example a catheter or electrode and, the instrumental guide 2, for example a Tuohy needle), a surgeon performs local anaesthesia. To the connector 19 of the needle is connected the claimed device with the aid of the cone 20 of the first stationary tube 8.

The medical instrument 4 (catheter, electrode etc.) is mounted via the distal portion of the movable tube 9, i.e. in the direction from a "caudal" portion to a "cephalic" portion, in the device in an initial position, with the front (proximal) end of the medical instrument 4 mounted level with the needle end. The medical instrument 4 is checked for being reliably gripped by the medical instrument 4 preliminary fixation means 3 and also by the detent 11 by way of slightly pulling with the distal end of the catheter. The collar 15 is held in a required place ( 5 mm, 10 mm, 15 mm, 20 mm) with the aid of the adjusting screw 18 on the depth scale 7 thereby to regulate a depth of inserting the instrument 4.

The device is set in a working position by way of expanding the spring 10 behind the arresting device 12, with the double-arm spring-loaded lever 16 mounted on the latter and turned through around the hinge 17 when pressed.

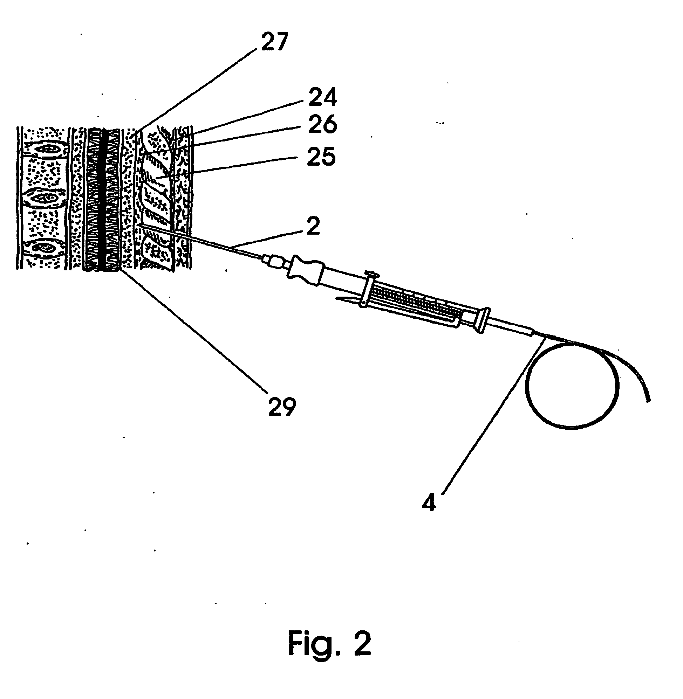

The needle 2 is used for puncturing soft tissues (as is the case with ordinary lumbar puncture) up to the level of a flaval ligament 26 (Fig.2). The surgeon establishes this moment accurately by a change in the density of passable tissues. The double-arm spring-loaded lever 16 is then pressed, one of the arms sliding with the surface 23 over the arresting device 12 thus releasing it for movement. Sequentially after passage of the dense tissues of a flaval ligament 26 and admission of the needle's end into the initial parts of an epidural space 27 , the spring 10 operates, and the second tube 9 moves inside the first tube 8. The arresting device 12 travels alongside the second tube 9 and the medical instrument 4, being present in the slot 13 of the body 1, and the tube 9 is in the end face guide 22. At the end of movement, the arresting device 12 interacts with the end face 14 of the slot 13.

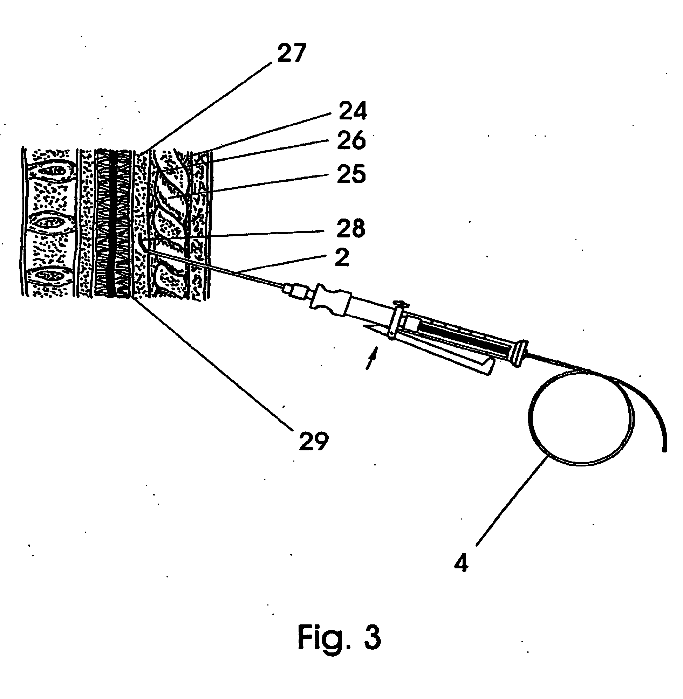

As a result, the automatic atraumatic movement of the medical instrument 4 (catheter, electrode, etc. ) is brought about into the epidural space 27 (in a proximal direction) for a regulated depth 28 (Fig. 3).

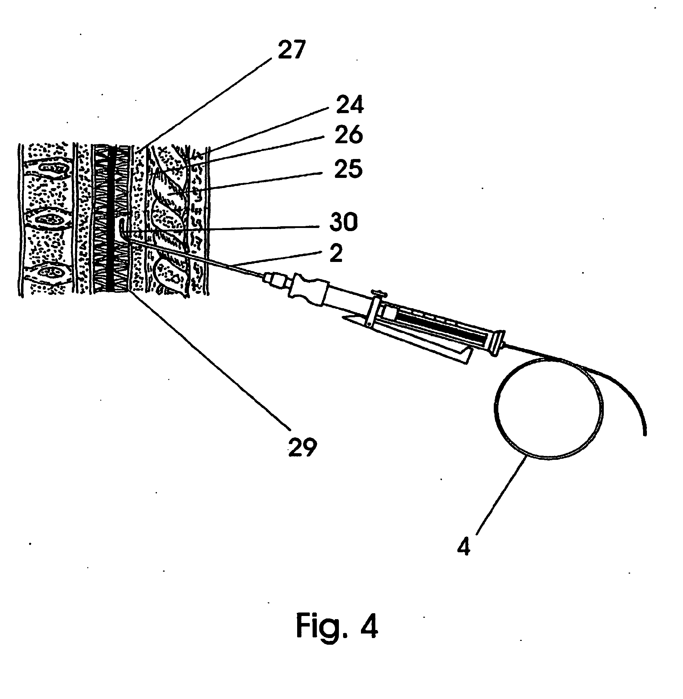

At the time of placing the medical instrument 4 (catheter) into the subdural space, a puncture is made in soft tissues, supraspinal 24, interspinal 25, flaval 26 ligaments, the epidural space 27 and after puncturing the dura mater 29 (with a characteristic click), the double-arm spring-loaded lever 16 is pressed, the spring 10 operates, the second tube 9 moves inside the first tube 8 alongside the medical instrument 4 (catheter) thereby to provide the automatic, atraumatic travel of the medical instrument 4 (catheter) into the subdural space (in a proximal direction) for a specified (regulated) depth 30 (Fig. 4).

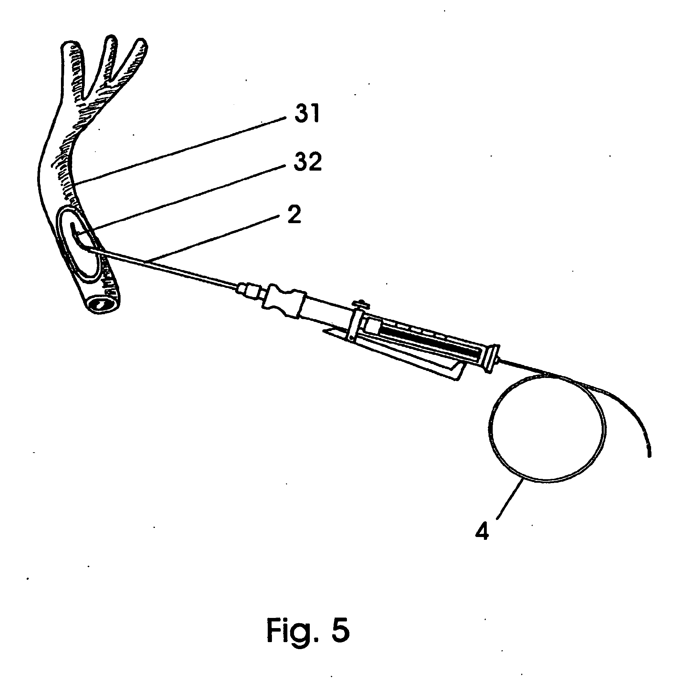

While placing the medical instrument 4 (catheter) in various vessels 31 (arteries, venae) and diverse hollow organs (joint cavity etc.) a puncture is made in soft tissues, the anterior wall of a vessel (artery, vena) or the anterior wall of a hollow organ followed by pressure on the double-arm spring-loaded lever 16, operation of the spring 10, travel of the second tube 9 inside the first tube 8 along with the medical instrument 4 (catheter) thereby to provide the automatic atraumatic movement of the medical instrument 4 (catheter) into a vessel cavity 32 or a hollow organ cavity (in a proximal direction) for a specified (regulated) depth. And while installing the medical instrument in the vessel lumen, the posterior vessel wall is not damaged, as is the case with traditional artery puncture technique, for example angiography (Fig. 5).

On completion of a procedure, a device with a needle and the medical instrument 4 (catheter , electrode etc.) is led out to take up an initial position. The medical instrument 4 (catheter, electrode etc.) is released from the device by pulling in the direction of insertion. To avoid a possible failure of the device, extraction of the medical instrument 4 (catheter, electrode etc.) backwards is not allowed, which is attributed to the construction of the detent 11 and that of the medical instrument preliminary fixation 3 means.

It is hence only logical to see that the medico-technical efficiency of the device, as sought for protection, consists in that it provides the automatic atraumatic installation of medical instruments (catheter, electrode etc.) into various spaces and hollow organs of a human body: the epidural or subdural space of the spinal cord, vessels (arteries, venae), into diverse hollow organs (joint cavity etc.) for a specified (regulated) depth and, along with this, the medical instruments are mounted into the epidural space of the spinal cord automatically right after making a puncture in the flaval ligament; the medical instruments are installed into the subdural space sequentially after puncture of the dura mater of the spinal cord while the installation of medical instruments in vessels and hollow organs is carried out after puncturing only one (anterior) wall of a vessel or hollow organ.

The claimed device can be used in various fields of medicine: neurosurgery, anaesthesiology, endovascular surgery, orthopedic surgery.

## Claims

1. Device for placing medical instruments, comprising a body (1) with an instrumental guide (2), a means (3) for preliminarily fixing a medical instrument (4), a trigger (6) with a depth scale (7) applied onto the body (1), and a means (5) for delivering the medical instrument (4) in the form of telescopic tubes (8, 9) arranged inside the body (1), of which one (8) is immovably coupled with the body (1) and the second one (9) is movable, spring-loaded, mounted with its end portion in a body's end face guide (22) and has a detent (11) for retaining the medical instrument (4) and an arresting device (12) provided in a body's slot (13) with freedom to move in a longitudinal direction and cooperate with the end face (14) of the slot (13), the trigger (6) being disposed on the body (1) with a faculty of travel, fixation and interaction with the detent (11) to set a depth of mounting said medical instruments (4),
**characterized in that** the minimum insertion depth of the movable tube (9) into the stationary tube (8) is at least one-half the outer diameter of the movable tube (9), and the distance between the end face guide (22) of the body (1) and the end portion oriented to said end face guide (22) of the stationary tube (8) is from 2 to 25 outer diameters of the movable tube (9).

2. Device according to claim 1, **characterized in that** the trigger (6) is configured as a collar (15) and a double-arm, spring-loaded lever (16) hinge (17) jointed with the collar (15), the latter has an adjusting screw (18), and one arm of the spring-loaded double-arm lever (16) has a surface (23) for cooperation with the detent (11).

3. Device according to claim 1 or 2, **characterized in that** the instrumental guide (2) is a hollow needle and connected with the body (1) via a connector (19) and a cone (20) being congruent thereto and provided on the stationary tube (8).

4. Device according to claim 3, **characterized in that** the instrumental guide (2) is configured as a Tuohy needle.

5. Device according to any one of claims 1 to 3,
**characterized in that** the body (1) is provided with a cap (21) arranged on its end, on the side of the instrumental guide (2) and retaining the medical instrument delivery means (5) in the body (1).

6. Device according to any one of claims 1 to 3 or 5, **characterized in that** the medical instrument preliminary fixation means (3) and the medical instrument retention detent (11) are embodied as spring grips of one-sided and/or double-sided action.

## Patentansprüche

1. Vorrichtung zum Platzieren von medizinischen Instrumenten, umfassend einen Körper (1) mit einer Instrumentenführung (2), ein Mittel (3) zum vorläufigen Befestigen eines medizinischen Instruments (4), einen Auslöser (6) mit einer Tiefenskala (7), die an den Körper (1) angelegt wird, und ein Mittel (5) zum Zuführen des medizinischen Instruments (4) in der Form von Teleskopröhren (8, 9), die innerhalb des Körpers (1) angeordnet sind, von denen eine (8) unbeweglich an den Körper (1) gekoppelt ist und die zweite (9) beweglich, federbeaufschlagt, mit ihrem Endabschnitt in einer Endflächenführung (22) des Körpers montiert ist und eine Rastung (11) zum Halten des medizinischen Instruments (4) und eine Arretierungsvorrichtung (12), die in einem Schlitz (13) des Körpers vorhanden ist, mit einer Freiheit, sich in einer Längsrichtung zu bewegen und mit der Endfläche (14) des Schlitzes (13) zusammenzuwirken, aufweist, wobei der Auslöser (6) an dem Körper (1) mit einer Fähigkeit zum Verfahren, Befestigen und mit der Rastung (11) Zusammenwirken, angeordnet ist, um eine Tiefe zum Montieren des medizinischen Instruments (4) einzustellen,
**dadurch gekennzeichnet, dass** die minimale Einführtiefe der beweglichen Röhre (9) in die stationäre Röhre (8) zumindest eine Hälfte des äußeren Durchmessers der beweglichen Röhre (9) ist und der Abstand zwischen der Endflächenführung (22) des Körpers (1) und dem Endabschnitt der stationären Röhre (8), der in Richtung der Endflächenführung (22) ausgerichtet ist, zwischen 2 und 25 äußeren Durchmessern der beweglichen Röhre (9) ist.

2. Vorrichtung nach Anspruch 1, **dadurch gekennzeichnet, dass** der Auslöser (6) als ein Stellring (15) und ein Doppelarm ausgeführt ist, der mit einem federbeaufschlagten Hebel (16) über ein Gelenk (17) mit dem Stellring (15) verbunden ist, wobei letzterer eine Einstellschraube (18) aufweist und ein Arm des federbeaufschlagten Doppelarmhebels (16) eine Oberfläche (23) zum Zusammenwirken mit der Rastung (11) aufweist.

3. Vorrichtung nach Anspruch 1 oder 2, **dadurch gekennzeichnet, dass** die Instrumentenführung (2) eine hohle Nadel ist und mit dem Körper (1) über einen Verbinder (19) und einen Kegel (20), der damit übereinstimmend und auf der stationären Röhre (8) vorhanden ist, verbunden ist.

4. Vorrichtung nach Anspruch 3, **dadurch gekennzeichnet, dass** die Instrumentenführung (2) als eine Tuohy-Nadel eingerichtet ist.

5. Vorrichtung nach einem der Ansprüche 1 bis 3, **dadurch gekennzeichnet, dass** der Körper (1) mit einer Kappe (21) versehen ist, die an seinem Ende an der Seite der Instrumentenführung (2) angeordnet ist und das Zuführmittel (5) für das medizinische Instrument in dem Körper (1) hält.

6. Vorrichtung nach einem der Ansprüche 1 bis 3 oder 5, **dadurch gekennzeichnet, dass** das Mittel (3) zum vorläufigen Befestigen des medizinischen Instruments und die Rastung (11) zum Halten des medizinischen Instruments als Federgreifer einer einseitigen und/oder zweiseitigen Wirkung ausgeführt sind.

## Revendications

1. Dispositif de mise en place d'instruments médicaux, comprenant un corps (1) avec un guide instrumental (2), des moyens (3) pour fixer au préalable un instrument médical (4), un déclencheur (6) avec une échelle de profondeur (7) appliqué sur le corps (1), et des moyens (5) pour mettre en place l'instrument médical (4) sous la forme de tubes télescopiques (8, 9) agencés à l'intérieur du corps (1), dont un (8) est couplé fixement au corps (1) et dont le second (9) est mobile, contraint par un ressort, monté avec sa partie d'extrémité dans un guide de face d'extrémité du corps (22) et présente un cliquet (11) adapté pour retenir l'instrument médical (4) et un dispositif d'arrêt (12) prévu dans une encoche du corps (13) ayant la liberté de se déplacer dans une direction longitudinale et de coopérer avec la face d'extrémité (14) de l'encoche (13), le déclencheur (6) étant disposé sur le corps (1) avec une faculté de déplacement, de fixation et d'interaction avec le cliquet (11) pour définir une profondeur d'introduction desdits instruments médicaux (4),
**caractérisé en ce que** la profondeur d'insertion minimale du tube mobile (9) dans le tube fixe (8) est d'au moins la moitié du diamètre externe du tube mobile (9), et la distance entre le guide de face d'extrémité (22) du corps (1) et la partie d'extrémité du tube fixe (8) orientée vers ledit guide de face d'extrémité (22) est de 2 à 25 diamètres externes du tube mobile (9).

2. Dispositif selon la revendication 1, **caractérisé en ce que** le déclencheur (6) est configuré comme un collier (15) et un double bras, l'articulation (17) du levier contraint par un ressort (16) étant jointe au collier (15), ce dernier présentant une vis de réglage (18), et un bras du levier à double bras (16) contraint par un ressort présentant une surface (23) pour coopération avec le cliquet (11).

3. Dispositif selon la revendication 1 ou 2, **caractérisé en ce que** le guide instrumental (2) est une aiguille creuse et reliée au corps (1) via un connecteur (19) et un cône (20) congruent à celui-ci et prévu sur le tube fixe (8).

4. Dispositif selon la revendication 3, **caractérisé en ce que** le guide instrumental (2) est configuré comme une aiguille de Tuohy.

5. Dispositif selon l'une quelconque des revendications 1 à 3, **caractérisé en ce que** le corps (1) est doté d'un capuchon (21) agencé sur son extrémité, sur le côté du guide instrumental (2) et retenant les moyens de mise en place de l'instrument médical (5) dans le corps (1).

6. Dispositif selon l'une quelconque des revendications 1 à 3 ou 5, **caractérisé en ce que** les moyens de fixation préalable de l'instrument médical (3) et le cliquet de rétention de l'instrument médical (11) sont intégrés sous la forme de poignées à ressort à action unilatérale et/ou bilatérale.
